# EUROPEAN PATENT SPECIFICATION

(11) **EP 3 099 661 B1**
(45) Date of publication and mention of the grant of the patent: **29.08.2018**
(21) Application number: 15702371.4
(22) Date of filing: 28.01.2015
(51) Int. Cl.: C07C 49/513, C07C 49/517

(54) **CLOSTRUBINS**
CLOSTRUBINE
CLOSTRUBINS

(30) Priority: 28.01.2014 EP 14000306; 03.02.2014 GB 201401807
(43) Date of publication of application: 07.12.2016
(73) Proprietor: Leibniz-Institut für Naturstoff-Forschung und Infektionsbiologie, 07745 Jena (DE)
(72) Inventor: PIDOT, Sacha, Victoria 3153 (AU); ISHIDA, Keishi, 07743 Jena (DE); SHABUER, Gulimila, 07747 Jena (DE); CYRULIES, Michael, 04288 Leipzig (DE); HERTWECK, Christian, 04105 Leipzig (DE)
(74) Representative: Forstmeyer, Dietmar
(86) International application number: PCT/EP2015/000162
(87) International publication number: WO 2015/113761

(56) References cited:
- WO-A1-2011/091778
- SCHOLZ ET AL.: "Chemie angeregter Zustände. V. Mitt.: Photocyclisierung von Benzalanthronen", TETRAHEDRON LETTERS, vol. 32, 1970, pages 2835-2838, XP002738197,
- PIDOT ET AL.: "Discovery of Clostrubin, an Exceptional Polyphenolic Polyketide Antibiotic from a Strictly Anaerobic Bacterium", ANGEW. CHEM., vol. 126, 14 May 2014 (2014-05-14), pages 7990-7993, XP002738198,

## Description

### Field of the invention

The present invention relates to a bioactive compound according to general formula (I); to a pharmaceutical composition comprising one or more of the compound(s); and to the compound for use as an antibiotic, cytotoxic and/or anti-proliferative agent.

### Background of the invention

In view of the rapid decline in the effectiveness of antibiotics due to the emergence of resistance, there is a need for a constant supply of new antibiotics for effective treatment of bacterial infections.

Similarly, the treatment of proliferative diseases such as cancer is one of the biggest medicinal challenges, since these diseases are one of the leading causes of death. Although much progress has been made in the development of anti-proliferative agents, there is a need for novel agents that help expanding the life expectancy of patients suffering from a proliferative disease.

Therefore, the problem underlying the present invention is to provide novel compounds having antibacterial, cytotoxic and/or anti-proliferative activity, especially compounds having antimicrobial activity against Gram-positive bacteria.

Scholz et al. (Tetrahedron Letters (32), 1970, pp. 2835-2838) disclose the photocyclisation of benzalanthrones resulting in naphto[1',3'; 1,9]anthrones.

WO 2011/091778 discloses the production and use of antibacterial, antiproliferative and antiphytopathogenic benzanthracenes.

### Summary and description of the invention

The present invention relates to a compound of the general formula (I): 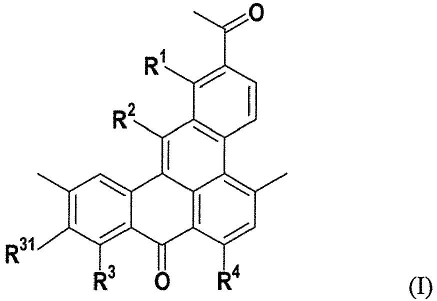 or a pharmacologically acceptable salt thereof, wherein
R¹, R², R³ and R⁴ each independently represents H, OH, F, Cl, CF₃, NH₂, -C(O)OC₁₋₆ alkyl, -CONH₂, -OC₁₋₆ alkyl, -O-C(O)C₁₋₆ alkyl, -NHC₁₋₆ alkyl, -NH-C(O)C₁₋₆ alkyl;-C(=O)NHC₁₋₆ alkyl; -NHSO₂C₁₋₆ alkyl; SOCH₃; SOCF₃; -SO₂NH₂, SO₂N(C₁₋₆ alkyl); or a group comprising a linear or branched polyethylene glycol (PEG) group which comprises the formula -(CH₂-CH₂-O)ₜ- with t being an integer of from 2 to 100 and has a molecular weight (MW) of from 400 to 50000 Da; and
R³¹ represents H or a group 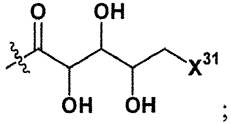 wherein X³¹ is OH or OC₁₋₃alkyl.

Compounds are usually described herein using standard nomenclature or the definitions presented below. For compounds having asymmetric centers, it should be understood that (unless otherwise specified) all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms.

Compounds according to the formulas provided herein, which have one or more stereogenic centers, have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example a chiral HPLC column.

Certain compounds are described herein using a general formula that includes variables, *e.g.* R¹, R², R³, and R⁴. Unless otherwise specified, each variable within such a formula is defined independently of any other variable, and any variable that occurs more than one time in a formula is defined independently at each occurrence. Thus, for example, if a group is shown to be substituted with 0-2 R*, the group may be unsubstituted or substituted with up to two R* groups and R* at each occurrence is selected independently from the definition of R*. Also, combinations of substituents and/or variables are permissible only if such combinations result in stable compounds, *i.e*., compounds that can be isolated, characterized and tested for biological activity.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range. For example, the term "C₁-C₆" refers to 1 to 6, *i.e.* 1, 2, 3, 4, 5 or 6, carbon atoms.

A "pharmacologically acceptable salt" of a compound disclosed herein is an acid or base salt that is generally considered in the art to be suitable for use in contact with the tissues of human beings or animals without excessive toxicity or carcinogenicity, and preferably without irritation, allergic response, or other problem or complication. Such pharmaceutical salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to - 4 (*i.e.,* 0, 1, 2, 3, or 4) and the like. Similarly, pharmaceutically acceptable cations include, but are not limited to sodium, potassium, calcium, aluminum, lithium and ammonium. Those of ordinary skill in the art will recognize further pharmacologically acceptable salts for the compounds provided herein. In general, a pharmacologically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

It will be apparent that each compound of formula (I) may, but need not, be present as a hydrate, solvate or non-covalent complex.

A "substituent," as used herein, refers to a molecular moiety that is covalently bonded to an atom within a molecule of interest. For example, a "ring substituent" may be a moiety such as a halogen, alkyl group, haloalkyl group, hydroxy, cyano, amino, nitro, mercapto, or other substituent described herein that is covalently bonded to an atom (preferably a carbon or nitrogen atom) that is a ring member. The term "substituted," as used herein, means that any one or more hydrogens on the designated atom is replaced with a selection from the indicated substituents, provided that the designated atom's normal valence is not exceeded, and that the substitution results in a stable compound, *i*.*e*. a compound that can be isolated, characterized and tested for biological activity. When a substituent is oxo (*i.e.,* =O), then 2 hydrogens on the atom are replaced. An oxo group that is a substituent of an aromatic carbon atom results in a conversion of -CH- to -C(=O)- and a loss of aromaticity. For example a pyridyl group substituted by oxo is a pyridone.

As used herein, "comprising", "including", "containing", "characterized by", and grammatical equivalents thereof are inclusive or open-ended terms that do not exclude additional, unrecited elements or method steps. "Comprising", etc. is to be interpreted as including the more restrictive term "consisting of".

As used herein, "consisting of' excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

The expression alkyl refers to a saturated, straight-chain or branched hydrocarbon group that contains from 1 to 20 carbon atoms, preferably from 1 to 12 carbon atoms, more preferably from 1 to 6 carbon atoms, for example a methyl, ethyl, propyl, iso-propyl, n-butyl, iso-butyl, *sec*-butyl, *tert*-butyl, n-pentyl, n-hexyl, 2,2-dimethylbutyl or n-octyl group.

The activity and more specifically the bioactivity of the compounds according to the present invention can be assessed using appropriate assays known to those skilled in the art, e.g. *in vitro* or *in vivo* assays. For instance, the antimicrobial activity may be determined using a standardized antibiotic assay^{[1]}, and the antiproliferative and/or cytotoxic activity may be determined via a standardized assay as described in reference [2].

In the compound of formula (I), R¹ and R² can each independently represent H, OH, F, Cl, CF₃, NH₂, -C(O)OC₁₋₆ alkyl, -CONH₂; -OC₁₋₆ alkyl; -NHC₁₋₆ alkyl; SOCH_{3;} SOCF₃; or -SO₂NH₂;
R³ or R⁴ can represent a group comprising a linear or branched polyethylene glycol (PEG) group which comprises the formula -(CH₂-CH₂-O)ₜ- with t being an integer of from 2 to 100 and has a molecular weight (MW) of from 400 to 50000 Da; and the other can be, independently of R¹, defined as R¹.

Further preferred is a compound of formula (I), or a pharmacologically acceptable salt thereof, wherein
R¹, R², R³ and R⁴ each independently represents OH, F, Cl, CF₃, NH₂, -C(O)OC₁₋₆ alkyl, -CONH₂, -OC₁₋₆ alkyl; SOCH₃; SOCF₃; or -SO₂NH₂.

Also preferred is a compound of formula (I), or a pharmacologically acceptable salt thereof, wherein R¹, R², R³ and R⁴ each independently represents OH, F, Cl, CF₃, NH₂, -OC₁₋₆ alkyl; or -SO₂NH₂.

The compound of formula (I) can be:

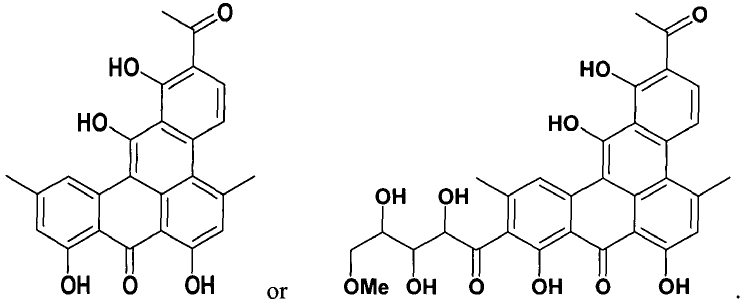

The present invention also relates to the use of the compound of formula (I) as active ingredient in the preparation or manufacture of a medicament.

A pharmaceutical composition according to the present invention comprises at least one compound of formula (I) and, optionally, one or more carrier substance(s), excipient(s) and/or adjuvant(s). Pharmaceutical compositions may additionally comprise, for example, one or more of water, buffers (*e.g.,* neutral buffered saline or phosphate buffered saline), ethanol, mineral oil, vegetable oil, dimethylsulfoxide, carbohydrates (*e.g.,* glucose, mannose, sucrose or dextrans), mannitol, proteins, adjuvants, polypeptides or amino acids such as glycine, antioxidants, chelating agents such as EDTA or glutathione and/or preservatives.

Furthermore, one or more other active ingredients may (but need not) be included in the pharmaceutical compositions provided herein. For instance, the compounds of the invention may advantageously be employed in combination with another antibiotic or antifungal agent, an anti-viral agent, an anti histamine, a non-steroidal anti-inflammatory drug, a disease modifying anti-rheumatic drug, another cytostatic drug, a drug with smooth muscle activity modulatory activity or mixtures of the aforementioned.

Pharmaceutical compositions may be formulated for any appropriate route of administration, including, for example, parenteral administration. The term parenteral as used herein includes subcutaneous, intradermal, intravascular such as, *e.g*., intravenous, intramuscular, spinal, intracranial, intrathecal, intraocular, periocular, intraorbital, intrasynovial and intraperitoneal injection, as well as any similar injection or infusion technique.

Carrier substances are, for example, cyclodextrins such as hydroxypropyl β-cyclodextrin, micelles or liposomes, excipients and/or adjuvants. Customary excipients include, for example, inert diluents such as, *e.g.,* calcium carbonate, sodium carbonate, lactose, calcium phosphate or sodium phosphate, granulating and disintegrating agents such as, *e.g.,* corn starch or alginic acid, binding agents such as, *e.g.,* starch, gelatin or acacia, and lubricating agents such as, *e.g.,* magnesium stearate or stearic acid. Examples of adjuvants are aluminum hydroxide, aluminum phosphate, calcium phosphate hydroxide, paraffin oil, squalene, thimerosal, detergents, Freund's complete adjuvant, or Freund's incomplete adjuvant.

For the prevention and/or treatment of bacterial infections, especially infections with Gram-positive bacteria, or proliferative diseases, the dose of the biologically active compound according to the invention may vary within wide limits and may be adjusted to individual requirements. Active compounds according to the present invention are generally administered in a therapeutically effective amount. The expression "therapeutically effective amount" denotes a quantity of the compound(s) that produces a result that in and of itself helps to ameliorate, heal, or cure the respective condition or disease. Preferred doses range from about 0.1 mg to about 140 mg per kilogram of body weight per day (about 0.5 mg to about 7 g per patient per day). The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination (*i*.*e*. other drugs being used to treat the patient) and the severity of the particular disease undergoing therapy.

Preferred compounds of the invention will have certain pharmacological properties. Such properties include, but are not limited to oral bioavailability, such that the preferred oral dosage forms discussed above can provide therapeutically effective levels of the compound *in vivo.*

The compound according to the invention as well as the pharmaceutical composition according to the invention can be used as a medicament, which can be administered to a patient (*e.g.* parenterally to a human or an other mammal), and will be present within at least one body fluid or tissue of the patient. As used herein, the term "treatment" encompasses both disease-modifying treatment and symptomatic treatment, either of which may be prophylactic, *i*.*e*., before the onset of symptoms, in order to prevent, delay or reduce the severity of symptoms, or therapeutic, *i.e.,* after the onset of symptoms, in order to reduce the severity and/or duration of symptoms. In particular, the conditions or diseases that can be ameliorated, prevented or treated using a compound of formula (I) or a pharmaceutical composition according to the invention include bacterial or fungal infections, or cancer. Within the present application the term "proliferative disease" encompasses disorders that are characterized by an overproduction (excessive proliferation) of a certain type of cells and turnover of cellular matrix, including cancer, atherosclerosis, rheumatoid arthritis, psoriasis, idiopathic pulmonary fibrosis, scleroderma and cirrhosis of the liver. For example, leukemia is a proliferative disorder characterized by an abnormal proliferation (overproduction) of white blood cells.

Within the present application the term "cancer" encompasses, but is not limited to, disorders, such as solid tumor cancer including breast cancer, lung cancer (non-small-cell lung cancer and small-cell lung cancer), prostate cancer, cancers of the oral cavity and pharynx (lip, tongue, mouth, pharynx), esophagus, stomach, small intestine, large intestine, colon, rectum, gallbladder and biliary passages, pancreas, larynx, lung, bone, osteosarcoma, connective tissue, skin cancer including Kaposi's syndrome, melanoma and skin metastasis, epidermoid cancer, basal cell carcinoma, cervix uteri, corpus endometrium, cancer of ovary, testis, bladder, ureter and urethra, kidney, eye, brain and central nervous system, pseudotumor cerebri, sarcoma, sarcoid, thyroid and other endocrine glands (including but not limited to carcinoid tumors), Hodgkin's disease, non-Hodkin's lymphomas, multiple myeloma, hematopoetic malignancies including leukemias and lymphomas including lymphocytic, granulocytic and monocytic lymphomas, tumor invasion, metastasis, ascites, tumor growth and angiogenesis.

Preferred compounds of the invention will have certain pharmacological properties. Such properties include, but are not limited to, bioavailability (especially with regard to oral administration), metabolic stability and sufficient solubility, such that the dosage forms can provide therapeutically effective levels of the compound *in vivo.*

The compounds of the present invention can be prepared in a number of ways well known to one skilled in the art of organic synthesis. The compounds of the present invention can be synthesized using methods known in the art of synthetic organic chemistry or by biotechnological approaches such as fermentation as appreciated by those skilled in the art. Since the structural complexity of the compounds precludes facile total synthetic access, semisynthetic as well as biotechnological approaches are commonly pursued in pharmaceutical research and development.

The following method for producing a compound of formula (I) also lies within the scope of the present invention. For instance, a compound of formula (I) can be produced by culturing *Clostridium beijerinckii* strain DSM13821. It is understood that the production of compounds of formula (I) is not limited to the use of the particular organism described herein, which is given for illustrative purpose only. The invention also includes the use of any mutants which are capable of producing a compound of formula (I) including natural mutants as well as artificial mutants, e.g. genetically manipulated mutants and the expression of the gene cluster responsible for biosynthesis in a producer strain or by heterologous expression in host strains.

A compound of formula (I) can be produced in liquid culture, by growing the respective microorganism under anaerobic conditions in media containing one or several different carbon sources, and one or different nitrogen sources. Also salts are essential for growth and production. Suitable carbon sources are different mono-, di-, and polysaccharides like maltose, glucose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions.

Temperatures for growth and production are between 25 °C to 40 °C, preferred temperatures are between 30 °C and 38.50 °C, especially at 37 °C. The pH of the culture solution is from 5 to 8, preferably a pH of 5.9 to 6.1, more preferably a pH of 6.0.

A compound of formula (I) can also be obtained by chemical synthesis in a number of ways well known to one skilled in the art of organic synthesis using usual chemical reaction and synthesis methods.

### Brief Description of the Figures

***Figure 1***. Structure of clostrubin A (**1**) and ³*J* (arrow) and ⁴*J* (dashed arrow) HMBC correlations. Observed enriched carbons from labeled acetate feeding experiments in ¹³C NMR spectra.

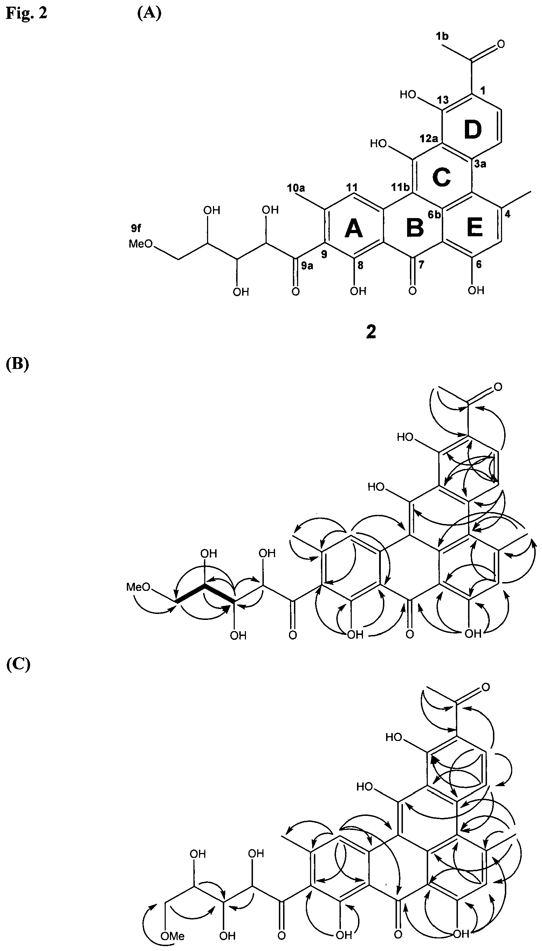

***Figure 2******.*** (**A**) Structure of clostrubin B (**2**). (**B**) Observed ¹H-¹H COSY (bold lines) and HMBC (arrows) correlations of **2** in DMSO-*d*₆. (**C**) Observed ¹H-¹H COSY (bold lines) and HMBC (arrows) correlations of **2** in DMF-*d*₇.

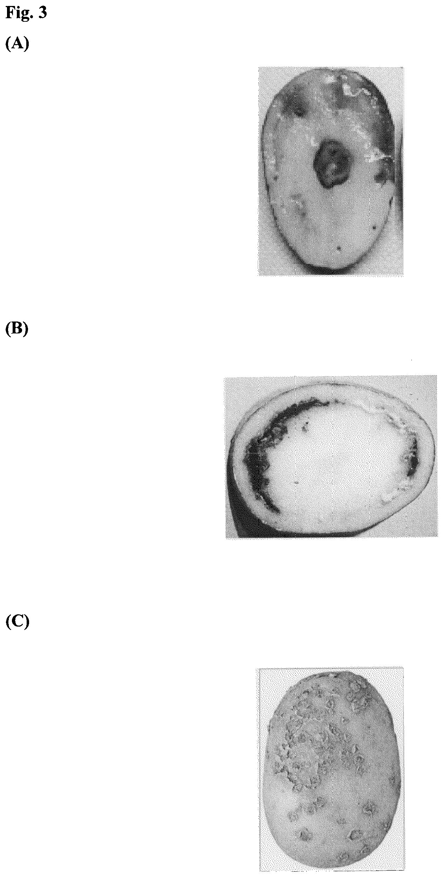

***Figure 3******.*** (**A**) Diseased potato showing soft rot symptoms. (**B**) Diseased potato showing ring rot symptoms. (**C**) Diseased potato showing scab symptoms.

The present invention is now further illustrated by the following examples from which further features, embodiments and advantages of the present invention may be taken.

### EXAMPLES

### General analytical procedures

NMR spectra were measured on Bruker Avance III™ 600 MHz spectrometer with cryo probe in DMSO-*d*₆. Spectra were referenced to the residual solvent peak. UV spectra were obtained on a Shimadzu UV-1800 spectrometer. A Jasco Fourier Transform Infrared Spectrometer was used to measure the infrared spectra using the ATR technique. LC-HRMS measurements were carried out on a Thermo Fisher Scientific Exactive Orbitrap with an electrospray ion source using a Betasil 100-3 C₁₈ column (150 × 2.1 mm) and an elution gradient [solvent A: H₂O + 0.1% HCOOH, solvent B: acetonitrile, gradient: 5% B for 1 min, 5% to 98% B in 15 min, 98% B for 15 min, flow rate: 0.2 mL min⁻¹, injection: 5 µL].

### Bacterial strains and culture conditions

*Clostridium beijerinckii* strain DSM13821 was grown in either 2 × YTG (16 g/L tryptone, 10 g/L yeast extract, 5 g/L NaCl and 5 g/L glucose) or P2 media anaerobically at 37 °C.

*C. beijerinckii* strain DSM13821 was grown in batch culture in volumes ranging from 500 mL to 4 L in Biostat Q fermenters (B. Braun Biotech International) using either 2YTG or P2 media. Anaerobic conditions were maintained by sparging fermenters with N₂ for at least 3 hours prior to culture inoculation and during each fermentation run. 5% culture volume of a 24 hr old culture in the same media was used to inoculate each fermenter. Fermentation cultures were incubated at 37 °C with stirring at 200 rpm and pH 6.0 was maintained by automatic addition of 10% NaOH or 2 M HCl.

### Screening for secondary metbolites

Small scale bacterial cultures were grown in 50 mL volumes for 48 hrs and subsequently extracted with an equal volume of ethyl acetate overnight. The two phases were separated and the ethyl acetate phase was dried over Na₂SO₄ and concentrated *in vacuo.* The residue was dissolved in 0.5 mL methanol and was analyzed via analytical HPLC (Shimadzu LC-10Avp series with autosampler, high pressure pumps, column oven and DAD detector, C18 column (Nucleosil 100-5C18, 120 × 4.6 mm), 0.8 mL/min flow rate, gradient elution (MeCN/0.1 % TFA 10/99.5 to 100/0 within 30 minutes).

### Example 1 Clostrubin A purification

Fermentation cultures (20 L) were initially extracted with equal volume of ethyl acetate overnight, after which the two phases were separated and the ethyl acetate phase was dried over Na₂SO₄ and then concentrated to dryness *in vacuo.* This extraction step, and all subsequent solvent extraction steps were performed three times each and subsequently combined. The dried phase was resuspended in 0.5M NaOH and then extracted with diethyl ether, which was subsequently removed and dried to give fraction 1 (F1). The pH of the aqueous phase was adjusted to 7.0 with HCl and again extracted with diethyl ether, which was then removed and dried to give F2. The aqueous phase was then acidified by the addition of HCl (to pH 2.0) and again extracted with diethyl ether, which was removed and dried to give F3. The acidic aqueous phase was subsequently extracted with ethyl acetate, which was removed and then dried, yielding F4. The aqueous phase was neutralized with NaOH (to pH 7.0) and again extracted with ethyl acetate, which was removed and dried yielding F5. Fraction F5 was then resuspended in 50% aqueous methanol, at which point particulates formed and the solution was filtered through a glass filter membrane. All particulate matter was removed from the filter by copious washing in MeOH, which was dried to completion. The sample was then analysed by HPLC and resuspended in DMSO-*d*₆ for NMR studies.

### Example 2 Biophysical characterisation of clostrubin A (1)

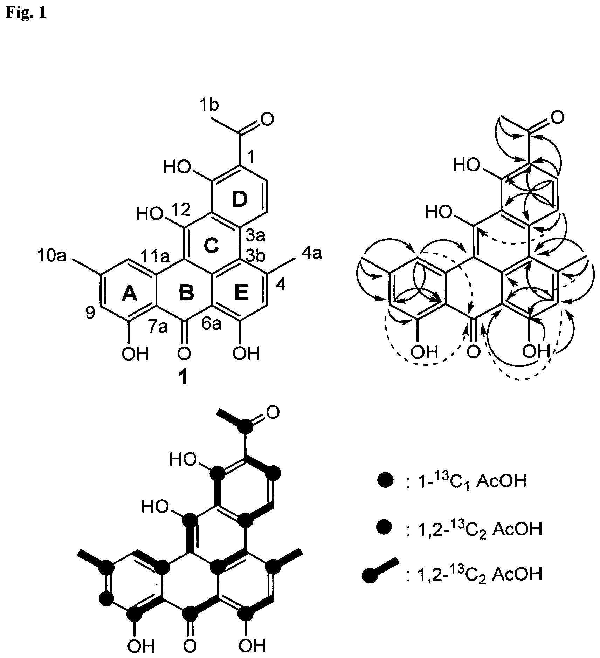

Orange powder. HRESIMS: [M-H]⁻ = 413.1030 (calculated for C₂₅H₁₇O₆ 413.1031); UV λₘₐₓ=, (DMSO): 303 (*ε* 76600), 377 (*ε* 29600), 416 (*ε* 14100), 551.0 (*ε* 63000) nm; (dioxane), 230 (*ε* 56000), 249 (*ε* 65100), 307 (*ε* 60300), 384 (*ε* 24700), 426 (*ε* 15000), 525 (*ε* 53600) nm; (MeOH), 203 (*ε* 88000), 248 (*ε* 44900), 300 (*ε* 41300), 377 (*ε* 14000), 416 (*ε* 7000), 542 (*ε* 2700) nm; (pyridine), 378 (*ε* 15400), 416 (*ε* 7300), 554 (*ε* 3200) nm; IR (ATR) : 1584, 1407, 1371, 1274, 1291, 1216, 1200, 997 cm⁻¹;
¹H and ¹³C NMR spectra of isolated purple material indicated that **1** was an aromatic polyketide, as there were limited proton signals and many aromatic carbons. Detailed analysis of ¹H NMR spectrum indicated the presence of three methyl, five aromatic, and two phenolic protons. ¹³C NMR and DEPT135 spectra revealed three methyl, five aromatic methine, and 17 quaternary carbons, including four phenolic and two carbonyl carbons. Extensive HMBC and HSQC analyses showed three ring systems; ring A and E which are di- and tri-substituted cresols, respectively, and ring D which is a di-substituted 1-(2-hydroxyphenyl)ethan-1-one (Figure 1). Ring A is connected to ring E by three ⁴*J* HMBC correlations from three singlet methine protons H5 (δ 6.80), H9 (δ 6.60), and H11 (δ 9.30) to keto carbonyl carbon C7 (δ 184.5). Two other ⁴*J* HMBC correlations from doublet methine proton H3 (δ 7.60) to phenolic carbon C12 (δ 178.0) and methyl proton H4a (δ 2.90) to quaternary carbon C6b (δ 133.6) suggested the presence of ring C. Finally, five aromatic rings were determined by two ³*J* HMBC correlations from H11 to C11b (δ 133.6) and H3 to C3b (δ 114.1). However, a quatenary carbon (C11a) was not assigned because of lacking HMBC correlations. To overcome this problem, cultures were grown in minimal media and supplemented with 1-¹³C or 1,2-¹³C labeled acetic acid, purified and analyzed by ¹³C NMR measurements. This process confirmed the presence of a number of quaternary carbons, whose correlations could now be reliably assigned to yield the final structure (**1**). The NMR data are summarized in Table 1 below.

**Table 1. NMR spectroscopic data of clostrubin A (1) (¹H and ¹³C in DMSO-d₆ at 300 K).**

| No. | ¹H (*J*, Hz) | ¹³C (mult) | 1,2-¹³C₂ AcOH (*J_{CC}*, Hz) | 1-¹³C AcOH Enriched |
|---|---|---|---|---|
| 1 | | 118.8 (s) | 58 | -^{§} |
| 1a | | 196.8 (s) | 42 | +^{§} |
| 1b | 2.68 (s) | 32.0 (q) | 42 | - |
| 2 | 7.80 (d 1.8) | 129.2 (d) | 58 | + |
| 3 | 7.60 (d 1.8) | 114.9 (d) | 56 | - |
| 3a | | 138.4 (s) | 56 | + |
| 3b | | 114.1 (s) | 55 | - |
| 4 | | 147.2 (s) | 41 | + |
| 4a | 2.90 (s) | 27.0 (q) | 41 | - |
| 5 | 6.80 (s) | 114.0 (d) | 66 | - |
| 6 | 14.88 (s, OH) | 164.5 (s) | 66 | + |
| 6a | | 107.3 (s) | 58 | - |
| 6b | | 133.6 (s) | 55 | + |
| 7 | | 184.7 (s) | 58 | + |
| 7a | | 111.2 (s) | 62 | - |
| 8 | 13.70 (s, OH) | 161.6 (s) | 62 | + |
| 9 | 6.60(s) | 110.6 (s) | + | - |
| 10 | | 145.0 (s) | 43 | + |
| 10a | 2.42 (s) | 22.8 (q) | 43 | - |
| 11 | 9.30 (s) | 117.6(d) | 56 | + |
| 11a | | 138.6 (s) | 56 | - |
| 11b | | 104.3 (s) | 66 | + |
| 12 | | 177.9 (s) | 66 | - |
| 12a | | 117.3 (s) | 59 | + |
| 13 | | 167.4 (s) | 59 | + |
| | | 167.4 (s) | 59 | + |

### Example 3 Assessment of antimicrobial activity

The antibiotic activity of clostrubin A was tested using a standardized antibiotic assay^{[1]}. Clostrubin A (**1**) was found to be ineffective against the Gram-negative organism, *Escherichia coli.* However, **1** was shown to be superior to ciprofloxacin (Cip) at inhibiting the growth of several Gram-positive organisms, including the notorious hospital pathogens methicillin-resistant *Staphylococcus aureus* (MRSA) and vancomycin-resistant enterococci (VRE), with a minimum inhibitory concentration (MIC) of 0.05 µg/ml (0.12 µM) and 0.4 µg/ml (0.97 µM), respectively (**Table 2**). A similar antimicrobial activity as against MRSA was observed against *Mycobacterium smegmatis* (*M. smegmatis*), *Mycobacterium aurum* (*M. aurum*), *Mycobacterium vaccae* (*M. vaccae*) and *Mycobacterium fortuitum* (*M. fortuitum*) - see, **Table 3.**

**Table 2. Antibacterial activity data for 1 and ciprofloxacin (cip).**

| Compound | | MIC (µM) | | |
|---|---|---|---|---|
| | Ec | Bs | MRSA | VRE |
| 1 | > 60.0 | 0.075 | 0.12 | 0.97 |
| Cip | 0.010 | 0.080 | 37 | 2.3 |

**Table 3. Anti-mycobacterial activity data for 1 and ciprofloxacin (cip).**

| Compound | MIC (µM) | | | |
|---|---|---|---|---|
| | *M. smegmatis* | *M. aurum* | *M. vaccae* | *M. fortuitum* |
| 1 | 0.48 | 0.12 | 0.12 | 0.12 |
| Cip | 4.71 | 0.30 | 1.20 | 0.60 |

### Example 4 Assessment of cytotoxic and anti-proliferative activity

Antiproliferative and cytotoxic activity of clostrubin A was tested in a standardized assay^{[2]}. The results are shown in **Table 4.**

**Table 4. Antiproliferative and cytotoxic activities of clostrubin A (1).**

| | Antiproliferative Effect | | Cytotoxicity |
|---|---|---|---|
| | HUVEC GI₅₀ | K-562 GI₅₀ | HeLa CC₅₀ |
| 1 | 1.20 µM | 2.66 µM | 1.69 µM |

As can be taken from the above results, clostrubin A has moderate antiproliferative and cytotoxic activity.

### Example 5 Isolation of clostrubin B

A fermentation culture of *C. beijerinckii* strain DSM13821 (8 L; P2 medium) was extracted with EtOAc/dioxane (4 times) and the extracts were dried over sodium sulfate. After the solvent was removed under reduced pressure, the residue was dissolved in 2-propanol and then filtered through a glass filter. The precipitate was dissolved in DMSO and subjected to RP-HPLC (Nucleosil 100-5C18, 10 × 125 mm, flow rate 3.5 mL/min) using a gradient system: solvent A (H₂O containing 0.1% TFA); solvent B (acetonitrile); 30%B for 10 min, to 100%B for 35 min, to keep for 10 min, to yield 1.5 mg of clostrubin B.

### Example 6 Biophysical characterisation of clostrubin B (2)

Orange powder. HRESIMS: [M-H]⁻ = 575.1570 (calculated for C₃₁H₂₇O₁₁ 575.1559); UV (DMSO) λₘₐₓ=288 (ε 16100), 303 (ε 16200), 379 (ε 6300), 420 (ε 4000), 555 (ε 12100) ; IR (ATR) : 2924, 2855, 1576, 1456, 1374, 1234, 1059, 1023 cm⁻¹.

Although clostrubin B (**2**) has a similar UV spectrum to clostrubin A (**1**), HR-MS analysis revealed a molecular fomula of C₃₁H₂₈O₁₁ for **2**, which is different from that of **1**. The ¹H and ¹³C NMR specra of **2** in DMSO-*d*₆ indicated that the ring structure B-E is almost the same as that of **1.** HSQC spectrum showed that methyl protons H10a (δ 2.42) and an aromatic proton H9 (δ 6.60) in A ring of **1** are disappeared in **2.** However the HMBC correlation H11 (δ 9.36) to C10a (δ 22.2) revealed the presence of methyl group. Thus the structure of **2** was suggested to be modified at the position C9 (δ118.9). The elongated part at C9, which has a sugar feature from C9b to C9f, was confirmed by ¹H-¹H COSY (H9c to H9e) and HMBC (H9f/C9e, H9c/C9b, C9d, C9e) correlations. Because the HMBC correlations H11/C11a and H9b,c/C9a were not observed, NMR spectra of **2** were measured in DMF-*d*₇. The chemical shift at C11a was assigned as 138.6 ppm by the HMBC correlation H11 to C11a. In the HSQC spectrum of **2** in DMF-*d*₇, a correlation between an unusual broad methyl proton at H10a and C10a was also observed. However one carbonyl signal at C9a was not observed in both solvent conditions. Either weak or broad signals at positions 7a, 8, 9, 10, 10a, 11, and 11a strongly indicated that a ring A structure in **2** may be tautomerized in both DMSO and DMF. Thus a carbon signal at C9a was not observed. The NMR data are summarized in Table 5 below.

**Table 5. NMR spectroscopic data of clostrubin B (2) (¹H and ¹³C in DMF-d₇ and DMSO-d₆, respectively; at 300 K).**

| DMF-*d*₇ | | | DMSO-*d*₆ | |
|---|---|---|---|---|
| No. | ¹H (*J*, Hz) | ¹³C (mult) | ¹H (*J*, Hz) | ¹³C (mult) |
| 1 | | 119.6 (s) | | 118.9 (s) |
| 1a | | 197.0 (s) | | 196.6 (s) |
| 1b | 2.74 (s) | 32.1 (q) | 2.67 (s) | 32.1 (q) |
| 2 | 7.93 (d 9.0) | 129.6 (d) | 7.80 (d 9.0) | 129.2 (d) |
| 3 | 7.66 (d 9.0) | 115.3 (d) | 7.58 (d 9.0) | 114.9 (d) |
| 3a | | 139.4 (s) | | 138.4 (s) |
| 3b | | 114.9 (s) | | 114.1 (s) |
| 4 | | 147.6 (s) | | 147.1 (s) |
| 4a | 2.99 (s) | 27.2 (q) | 2.92 (s) | 27.0 (q) |
| 5 | 6.82 (s) | 114.3 (d) | 6.79 (s) | 113.9 (d) |
| 6 | 15.00 (s, OH) | 165.5 (s) | 14.88 (s, OH) | 164.4 (s) |
| 6a | | 108.2 (s) | | 107.3 (s) |
| 6b | | 134.5 (s) | | 133.5 (s) |
| 7 | | 185.7 (s) | | 184.7 (s) |
| 7a | | 112.1 (s) | | 111.0 (s) |
| 8 | 14.53 (s, OH) | 161.7 (s) | 14.35 (s, OH) | 160.3 (s) |
| 9 | | 118.8 (s) | | 118.9 (s) |
| 9a | | - | | - |
| 9b | 3.41 (m) | 82.3 (d) | 3.21 (m) | 81.5 (d) |
| 9c | 3.48 (t 9.0) | 71.7 (d) | 3.19 (m) | 70.5 (d) |
| 9d | 3.53 (br) | 80.1 (d) | 3.47 (m) | 79.0 (d) |
| 9e | 3.68 (dd 12.1, 6.9) | 62.8 (t) | 3.44 (m) | 61.8 (t) |
| | 3.89 (dd 12.1, 2.1) | | 3.72 (brd 11.2) | |
| 9f | 3.76 (s) | 60.2 (q) | 3.47 (s) | 59.2 (q) |
| 10 | | 145.5(s) | | 145.0 (s) |
| 10a | 2.67 (br) | 22.2 (q) | 2.60 (br) | 22.2 (q) |
| 11 | 9.56 (s) | 120.0 (d) | 9.36 (s) | 119.5 (s) |
| 11a | | 138.6 (s) | | 137.6 (s) |
| 11b | | 105.0 (s) | | 104.3 (s) |
| 12 | | 179.1 (s) | | 178.0 (s) |
| 12a | | 118.3 (s) | | 117.4 (s) |
| 13 | | 168.6 (s) | | 167.7 (s) |

### Example 7 Activity against potato pathogens

The antimicrobial activity of clostrubin A (**1**) and clostrubin B (**2**) against the serious potato pathogens *Bacillus pumilus* (causing potato soft rot), *Clavibacter michiganensis ssp. sepedonicus* (causing potato ring rot), and *Streptomyces scabies* (causing potato scab) was tested. Both compounds were found to be more effective against these pathogens than ciprofloxacin. The results are shown in **Table 6,** where the minimum inhibitory concentration (MIC) for **1, 2,** and ciprofloxacin against the above potato pathogens is indicated.

**Table 6. Activity against potato pathogens**

| | **MIC (µM)** | | |
|---|---|---|---|
| | ***Bacillus pumilus*** | ***Clavibacter michiganensis ssp. sepedonicus*** | ***Streptomyces scabies*** |
| **Clostrubin A** | **0,047** | **0,0947** | **0,0947** |
| **Ciprofloxacin** | **0,81** | **3,24** | **1,620** |
| **Clostrubin B** | **0,542** | **0,542** | **0,271** |

### References

[1] I. Wiegand, K. Hilpert, R. E. Hancock, Nat. Protoc. 2008, 3, 163-175.
[2] M. Ziehl, J. He, H. M. Dahse, C. Hertweck, Angew. Chem., Int. Ed. 2005, 44, 1202-1205.

## Claims

1. A compound of the general formula (I): 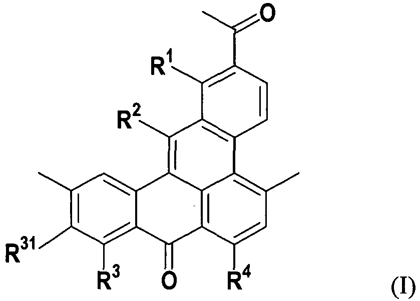 or a pharmacologically acceptable salt thereof, wherein
R¹, R², R³ and R⁴ each independently represents H, OH, F, Cl, CF₃, NH₂, -C(O)OC₁₋₆ alkyl, -CONH₂, -OC₁₋₆ alkyl, -O-C(O)C₁₋₆ alkyl, -NHC₁₋₆ alkyl, -NH-C(O)C₁₋₆ alkyl;-C(=O)NHC₁₋₆ alkyl; -NHSO₂C₁₋₆ alkyl; SOCH₃; SOCF₃; -SO₂NH₂, SO₂N(C₁₋₆ alkyl); or a group comprising a linear or branched polyethylene glycol (PEG) group which comprises the formula -(CH₂-CH₂-O)ₜ- with t being an integer of from 2 to 100 and has a molecular weight (MW) of from 400 to 50000 Da; and
R³¹ represents H or a group 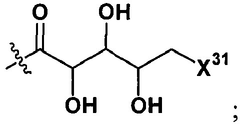 wherein X³¹ is OH or OC₁₋₃alkyl.

2. The compound according to claim 1, or a pharmacologically acceptable salt thereof, wherein
R¹ and R² each independently represents H, OH, F, Cl, CF₃, NH₂, -C(O)OC₁₋₆ alkyl, -CONH₂; -OC₁₋₆ alkyl; -NHC₁₋₆ alkyl; SOCH₃; SOCF₃; or -SO₂NH₂; and
R³ or R⁴ represents a group comprising a linear or branched polyethylene glycol (PEG) group which comprises the formula -(CH₂-CH₂-O)ₜ- with t being an integer of from 2 to 100 and has a molecular weight (MW) of from 400 to 50000 Da; and the other is, independently of R¹, defined as R¹.

3. The compound according to claim 1, or a pharmacologically acceptable salt thereof, wherein
R¹, R², R³ and R⁴ each independently represents OH, F, Cl, CF₃, NH₂, -C(O)OC₁₋₆ alkyl, -CONH₂, -OC₁₋₆ alkyl; SOCH₃; SOCF₃; or -SO₂NH₂.

4. The compound according to claim 1 or 3, or a pharmacologically acceptable salt thereof, wherein
R¹, R², R³ and R⁴ each independently represents OH, F, Cl, CF₃, NH₂, -OC₁₋₆ alkyl; or -SO₂NH₂.

5. The compound according to any one of claims 1, 3 and 4, wherein the compound is:

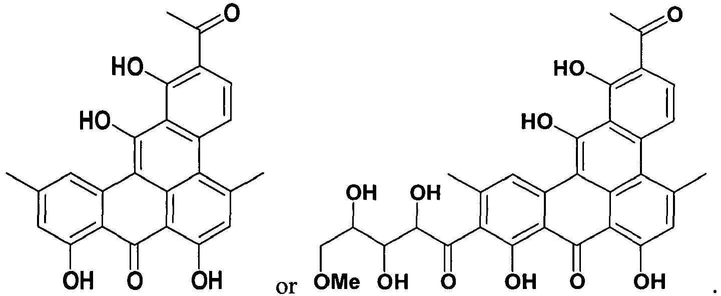

6. A pharmaceutical composition comprising at least one compound according to any one of claims 1 to 5 and, optionally, one or more carrier substance(s), excipient(s) and/or adjuvant(s).

7. The compound according to any one of claims 1 to 5, or the pharmaceutical composition according to claim 6, for use as a medicament.

8. The compound according to any one of claims 1 to 5, or the pharmaceutical composition according to claim 6, for use in the prevention and/or treatment of a bacterial infection.

9. The compound or the pharmaceutical composition for use as in claim 8, wherein the bacterial infection is caused by a Gram-positive microbe.

10. The compound according to any one of claims 1 to 5, or the pharmaceutical composition according to claim 6, for use in the prevention and/or treatment of a proliferative disease.

11. A method for the preparation of a compound of formula (I), the method comprising the steps of:
(a) culturing Clostridium beijerinckii strain deposited under DSM 13821; and
(b) separating and retaining the compound from the culture broth, the step comprising precipitation on a glass filter.

## Patentansprüche

1. Verbindung der allgemeinen Formel (I): 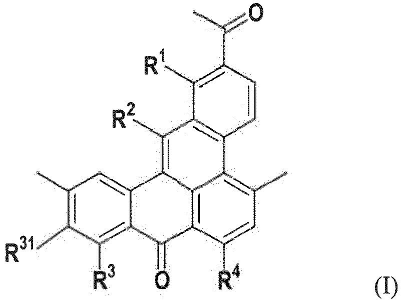 oder ein pharmakologisch akzeptables Salz derselben, wobei
R¹, R², R³ und R⁴ jeweils unabhängig voneinander H, OH, F, Cl, CF₃, NH₂, -C(O)OC₁₋₆ Alkyl, -CONH₂, -OC₁₋₆ Alkyl, -O-C(O)C₁₋₆ Alkyl, -NHC₁₋₆ Alkyl, -NH-C(O)C₁₋₆ Alkyl;-C(=O)NHC₁₋₆ Alkyl; -NHSO₂C₁₋₆ Alkyl; SOCH₃; SOCF₃; -SO₂NH₂, SO₂N(C₁₋₆ Alkyl) darstellt; oder eine Gruppe, umfassend eine lineare oder verzweigte Polyethylenglycol (PEG)-Gruppe, welche die Formel -(CH₂-CH₂-O)ₜ-, wobei t eine ganze Zahl von 2 bis 100 ist, umfasst und ein Molekulargewicht (MW) von 400 bis 50000 Da aufweist; und
R³¹ H oder eine Gruppe 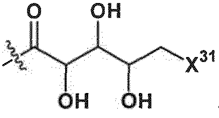 darstellt; wobei X³¹ OH oder OC₁₋₃ Alkyl ist.

2. Die Verbindung nach Anspruch 1, oder ein pharmakologisch akzeptables Salz derselben, wobei
R¹ und R² jeweils unabhängig voneinander H, OH, F, Cl, CF₃, NH₂, -C(O)OC₁₋₆ Alkyl, -CONH₂; -OC₁₋₆ Alkyl; -NHC₁₋₆ Alkyl; SOCH₃; SOCF₃; oder -SO₂NH₂ darstellt; und
R³ oder R⁴ eine Gruppe, umfassend eine lineare oder verzweigte Polyethylenglycol (PEG)- Gruppe, welche die Formel -(CH₂-CH₂-O)ₜ-, wobei t eine ganze Zahl von 2 bis 100 ist, umfasst und ein Molekulargewicht (MW) von 400 bis 50000 Da aufweist, darstellt; und der jeweils andere Rest, unabhängig von R¹, wie R¹ definiert ist.

3. Die Verbindung nach Anspruch 1, oder ein pharmakologisch akzeptables Salz derselben, wobei
R¹, R², R³ und R⁴ jeweils unabhängig voneinander OH, F, Cl, CF₃, NH₂, -C(O)OC₁₋₆ Alkyl, -CONH₂, -OC₁₋₆ Alkyl; SOCH₃; SOCF₃; oder -SO₂NH₂ darstellt.

4. Die Verbindung nach Anspruch 1 oder 3, oder ein pharmakologisch akzeptables Salz derselben, wobei
R¹, R², R³ und R⁴ jeweils unabhängig voneinander OH, F, Cl, CF₃, NH₂, -OC₁₋₆ Alkyl; oder -SO₂NH₂ darstellt.

5. Die Verbindung nach einem der Ansprüche 1, 3 und 4, wobei die Verbindung: 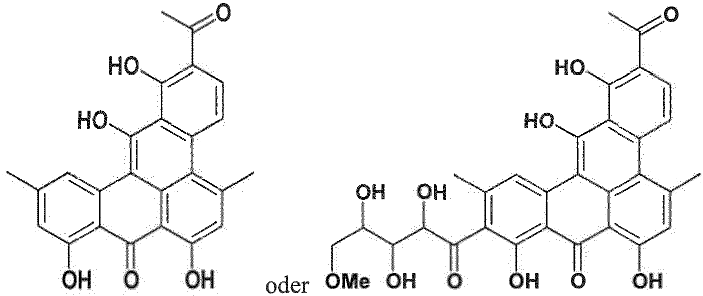 ist.

6. Pharmazeutische Zusammensetzung, die mindestens eine Verbindung gemäß einem der Ansprüche 1 bis 5 und gegebenenfalls eine oder mehrere Trägersubstanz(en), Arzneiträger und / oder Hilfsstoff(e) umfasst.

7. Die Verbindung gemäß einem der Ansprüche 1 bis 5, oder die pharmazeutische Zusammensetzung nach Anspruch 6, zur Verwendung als Medikament.

8. Die Verbindung gemäß einem der Ansprüche 1 bis 5, oder die pharmazeutische Zusammensetzung nach Anspruch 6, zur Verwendung bei der Vorbeugung und / oder Behandlung einer bakteriellen Infektion.

9. Die Verbindung oder die pharmazeutische Zusammensetzung zur Verwendung gemäß Anspruch 8, wobei die bakterielle Infektion durch eine Gram-positive Mikrobe verursacht ist.

10. Die Verbindung gemäß einem der Ansprüche 1 bis 5, oder die pharmazeutische Zusammensetzung nach Anspruch 6, zur Verwendung bei der Vorbeugung und / oder Behandlung einer proliferativen Erkrankung.

11. Verfahren zur Herstellung einer Verbindung der Formel (I), wobei das Verfahren die Schritte umfasst:
(a) das Kultivieren des Stamms *Clostridium beijerinckii,* der unter der Hinterlegungsnummer DSM 13821 deponiert wurde; und
(b) das Abtrennen und Gewinnen der Verbindung aus dem Kulturmedium; wobei der Schritt eine Ausfällung auf einem Glasfilter umfasst.

## Revendications

1. Composé de formule générale (I) : 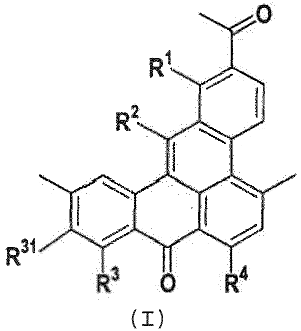 ou son sel pharmaceutiquement acceptable,
dans lequel
chacun de R¹, R², R³ et R⁴ représente indépendamment H, OH, F, Cl, CF₃, NH₂, -C(O)O-alkyle en C₁ à C₆, -CONH₂, -O-alkyle en C₁ à C₆, -O-C(O)-alkyle en C₁ à C₆, -NH-alkyle en C₁ à C₆, -NH-C(O)-alkyle en C₁ à C₆ ; -C (=O) NH-alkyle en C₁ à C₆ ; -NHSO₂-alkyle en C₁ à C₆ ; SOCH₃ ; SOCF₃ ; -SO₂NH₂, SO₂N-alkyle en C₁ à C₆ ; ou un groupe comprenant un groupe polyéthylèneglycol (PEG) linéaire ou ramifié qui comprend la formule -(CH₂-CH₂-O-)ₜ- où t est un entier de 2 à 100 et qui a une masse moléculaire (MW) de 400 à 50 000 Da ; et
R³¹ représente H ou un groupe 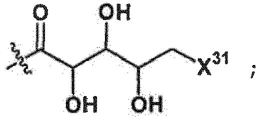 où X³¹ est OH ou O-alkyle en C₁ à C₃.

2. Composé selon la revendication 1, ou un sel pharmaceutiquement acceptable de celui-ci, dans lequel
chacun de R¹ et R² représente indépendamment H, OH, F, Cl, CF₃, NH₂, -C(O)O-alkyle en C₁ à C₆, -CONH₂ ; -O-alkyle en C₁ à C₆ ; -NH-alkyle en C₁ à C₆ ; SOCH₃ ; SOCF₃ ; ou -SO₂NH₂ ; et
R³ ou R⁴ représente un groupe comprenant un groupe polyéthylèneglycol (PEG) linéaire ou ramifié qui comprend la formule -(CH₂-CH₂-O-)ₜ- où t est un entier de 2 à 100 et qui a une masse moléculaire (MW) de 400 à 50 000 Da ; et l'autre, est, indépendamment de R¹, défini comme pour R¹.

3. Composé selon la revendication 1, ou un sel pharmaceutiquement acceptable de celui-ci, dans lequel
chacun de R¹, R², R³ et R⁴ représente indépendamment OH, F, Cl, CF₃, NH₂, -C(O)-alkyle en C₁ à C₆, -CONH₂, -O-alkyle en C₁ à C₆ ; SOCF₃ ; ou -SO₂NH₂.

4. Composé selon la revendication 1 ou 3, ou un sel pharmaceutiquement acceptable de celui-ci, dans lequel
chacun de R¹, R², R³ et R⁴ représente indépendamment OH, F, Cl, CF₃, NH₂, -C(O)-alkyle en C₁ à C₆ ; ou -SO₂NH₂.

5. Composé selon l'une quelconque des revendications 1, 3 et 4, lequel composé est :

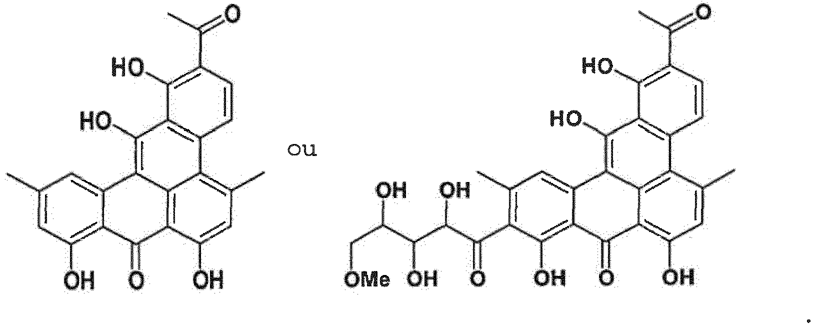

6. Composition pharmaceutique comprenant au moins un composé selon l'une quelconque des revendications 1 à 5 et éventuellement un ou plusieurs adjuvant(s), excipient(s) et/ou substance(s) de support.

7. Composé selon l'une quelconque des revendications 1 à 5, ou composition pharmaceutique selon la revendication 6, pour une utilisation en tant que médicament.

8. Composé selon l'une quelconque des revendications 1 à 5, ou composition pharmaceutique selon la revendication 6, pour une utilisation dans la prévention et/ou le traitement d'une infection bactérienne.

9. Composé ou composition pharmaceutique pour une utilisation selon la revendication 8, dans lequel l'infection bactérienne est provoquée par un microbe Gram positif.

10. Composé selon l'une quelconque des revendications 1 à 5, ou composition pharmaceutique selon la revendication 6, pour une utilisation dans la prévention et/ou le traitement d'une maladie proliférative.

11. Procédé pour la préparation d'un composé de formule (I), le procédé comprenant les étapes suivantes :
(a) culture de la souche Clostridium beijerinckii déposée sous la référence DSM 13821 ; et
(b) séparation et récupération du composé à partir du bouillon de culture, l'étape comprenant une précipitation sur un filtre en verre.
